# EUROPEAN PATENT APPLICATION

(11) **EP 2 695 999 A1**
(43) Date of publication of application: **12.02.2014**
(21) Application number: 12179534.8
(22) Date of filing: 07.08.2012
(51) Int. Cl.: E02F 3/26, E02F 9/26, E21C 39/00

(54) **An excavation system and a method of excavation**

(71) Applicant: Siemens Aktiengesellschaft, 80333 München (DE)
(72) Inventor: Das, Saptarshi, 560102 Bangalore (IN); Murugaiah, Thirumalai Kumar, 560099 Bangalore (IN)

(57) **Abstract**

The present invention discloses an excavation system (1) comprising:
- a controlling module (2) adapted to receive a sub-surface information (3) of an environment (17) being excavated by an excavator (16) of the excavation system (1), to process the sub-surface information (3) and to send a controlling signal (4) to a drive (5) of the excavator (16), and
- the drive (5) of the excavator (16) adapted to receive the controlling signal (4) from the controlling module (2) and to control the excavator (16) for excavation of the environment (17) on a basis of the controlling signal (4).

## Description

The invention relates to excavation of an environment. More specifically, the invention relates to excavation of the environment on a basis of sub-surface information of the environment.

In an open cast mining and other earth moving operations, for the purpose of excavations, utility of the excavators is indispensable. However, the excavators are also consumers of majority of the power consumptions during such operations. There is always a tradeoff between operational throughput and energy consumption. Generally advanced drive technology is used to strike this trade off. Moreover, the excavators are also required to be failsafe for seamless operations. In order to reduce the mechanical fatigue of the excavators, the speed of the excavation should be controlled as and when required, like according to the material that is being excavated. The physical property of the material also changes with respect to location of excavation, change in weather like humidity and rain that can make the material denser and heavier. Hence, it becomes challenging to manage excavation with respect to different materials at different locations and during different time period, weather conditions, etc.

The object of the invention is to provide a trade-off between throughput of excavation and the power utilized while keeping the excavator failsafe.

The object of the invention is achieved by an excavation system of claim 1 and a method for excavation of claim 9.

According to an embodiment of the excavation system, the excavation system includes a ground penetrating radar module and a processing module. The ground penetrating radar module sends a transmit signal to a sub-surface of the environment and receives a receive signal from the sub-surface and transfers the receive signal to a processing module. The processing module receives the receive signal and processes the receive signal for generating the sub-surface information. The excavation system also includes a controlling module and a drive of the excavator, wherein the controlling module receives the sub-surface information of the environment being excavated by the excavator of the excavation system and process the sub-surface information to send a controlling signal to the drive of the excavator, so that the drive of the excavator controls the excavator for excavation of the environment. Introduction of ground penetrating radar module in the excavation system allows the excavation system to generate the sub-surface information on the fly, while excavation of the environment is going on.

According to another embodiment of the excavation system, the ground penetrating radar module is physically coupled to the excavator. This helps to collect the sub-surface information of an area of the environment which is currently being excavated.

According to yet another embodiment of the invention, the system includes a material testing unit for testing material of the environment being excavated and provides a sub-surface test result to the processing module, so that the processing module processes the sub-surface test result in combination with the receive signal to generate the sub-surface information. This provides for an additional check to correct the sub-surface information being provided to the controlling module, so that excavation can be more accurate according to the sub-surface and the processing module can adjust if required.

According to one embodiment of the excavation system, the system includes a storage which receives the sub-surface information, stores the sub-surface information and provides the sub-surface information to the controlling module, as and when required.

According to another embodiment of the excavation system, wherein the controlling module receives a feedback from a motor of the excavator, processes the feedback in combination with the sub-surface information and generates the controlling signal. This help to provide additional controlling of the drive of the excavator, so that the excavation can be more efficient.

According to yet another embodiment of the excavation function, the controlling signal is at least related to one of a torque to be applied by the drive to the excavator and a frequency of rotation of excavator. Such controlling keeps a check of control either or both of the two ways, i.e., the torque and the frequency of rotation.

According to an exemplary embodiment of the excavation system, the excavator is a bucket wheel excavator. Using the bucket wheel excavator in the excavation system provides for the high throughput of the excavation.
- FIG 1: illustrates a schematic diagram of an excavation system,
- FIG 2: illustrates a bucket wheel excavator coupled to a ground penetrating module.

Hereinafter, various embodiments for carrying out the present invention are described in detail. The embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

Prior to explaining functioning of system through various embodiments, some of the terminology used herein will be explained.

"Excavator" is a powered machine designed to perform excavations for bulk handling operations like mining, digging earth, gravel, sand, etc in an environment.

"Environment" is a geographical area where the excavations are to be performed and includes surface, sub-surface, weather, etc. of the geographical area.

"Sub-surface" is defined by the location below or behind the surface of the environment.

"Controlling module", "Processing module" and "Material testing unit" are generally processors which are logic circuitry that responds to and processes the basic instructions for performing a function. They may be a central processing unit of a personal computer adapted to perform the function or microprocessors which are multipurpose, programmable devices that accepts data as input, processes it according to instructions stored in its memory, and provides results as output or any other computing device adapted to perform functions of the controlling module and/or the processing module and/or the material testing unit according to current invention. However, technical difference between the controlling module, the processing module and the material testing unit are explained through there functionalities while explaining the figures.

"Sub-surface information" is defined by a computerized data related to the sub-surface profile, specifically related to the physical properties like density of the sub-surface, moisture content, temperature, etc.

"Controlling signal" has electrical or mechanical property through the controlling module to activate or enable a drive of the excavator, so that the motor of the excavator can be controlled by the drive.

"Ground penetrating radar module" is a nondestructive geophysical device that produces cross-sectional profile or record of sub-surface features, without drilling, probing, or digging. Ground penetrating radar module operates by transmitting electromagnetic signals down into the ground through a transducer. The transmitted energy is reflected from various buried objects or distinct contacts between different earth materials. The antenna of the Ground penetrating radar module then receives the receive signals, produced due to reflection by buried objects or difference of material property.

"Material test result" is computerized data relating to material in the sub-surface profile.

"Storage" is an electronic storing medium which stores computerized and/or electronic data. Some examples of storage are CD ROM, RAM, Hard Disk, USB device, electromagnetic storing medium, optical storing medium or any other storage medium capable of storing computerized and/or electronic data.

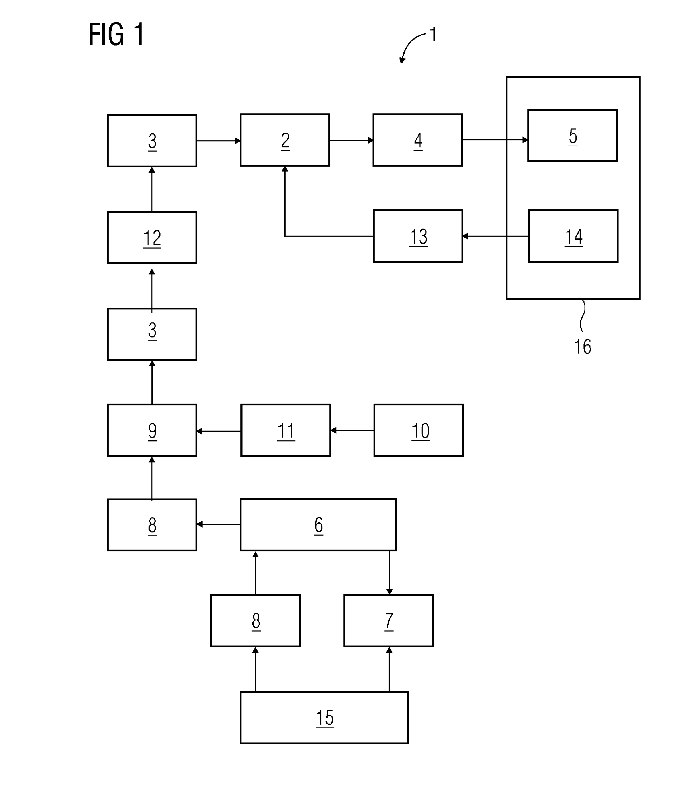

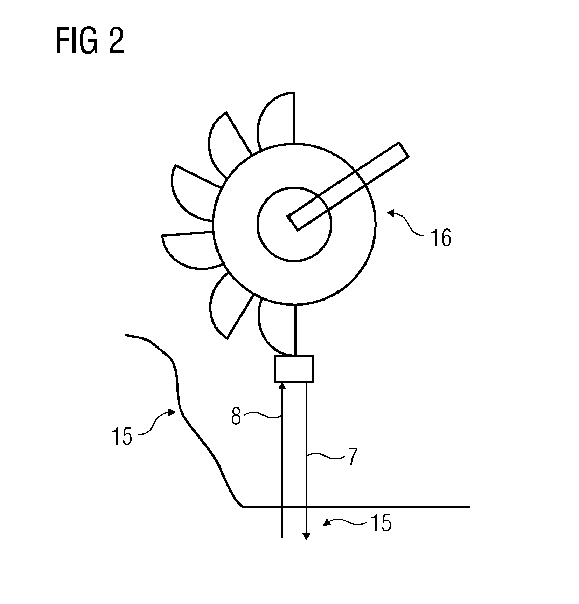

While discussing FIG 1 and FIG 2, references will be made to each other.

FIG 1 shows a controlling module 2, an excavator 16, a ground penetrating module 6, a processing module 9, a material testing unit 10 coupled together to establish the excavation system.

The excavator 16 comprises of a drive 5 and a motor 14 for excavation functionality. The drive 5 on receiving direction by a user of the excavator 16 drives the motor 14 at variable speed.

The controlling module 2 receives and processes sub-surface information 3 of a sub-surface 15 of an environment 17 being excavated by the excavator 16. On a basis of the processing, the controlling module 2 sends a controlling signal 4 to a drive 5 of the excavator 16. The drive 5 controls the excavator 16 for excavation of the environment on a basis of the controlling signal 4. The controlling module 2 also receives a feedback 13 from a motor 14 of the excavator 16 and processes the feedback 13 in combination with the sub-surface information 3 and to generate the controlling signal 4. In an alternate embodiment, the controlling module 2 need not receive the feedback from the motor 14 and processes the sub-surface information alone to generate the controlling signal 4. In yet another embodiment, the controlling module 2 processes the feedback and the sub-surface information 3 independently to produce two different signals for controlling excavations, wherein the two different signals in combination forms the controlling signal 4. In one embodiment, the controlling module 2 also receives environmental information like power cuts, etc. to be processed in combination with the sub-surface information 3 and the feedback 13 to produce the controlling signal 4 or to be processed independently to produce an independent signal which forms a component of the controlling signal 4.

The controlling module 2 receives the sub-surface information from a processing module 9 via storage 12. The storage 12 receives and stores the subsurface information produces by a processing module 9. However, in an alternate embodiment, the storage can receive the sub-surface information 3 from any other sources like weather forecasts, historical sub-surface data, etc. The sub-surface information 3 can be sent to the controlling module 2 by the storage 12 when queried by the controlling module 2 or by the storage 12 in a timely manner, or in any fashion as desired by the excavation system 1.

The processing module 9 receives a receive signal 8 from a ground penetrating radar module 6 and processes the receive signal 8 and generates the sub-surface information 3. The sub-surface information 3 so generated is further sent to the storage 12 by the processing module 9. In an alternate embodiment, the processing module 9 need not receive the receive signal 8 from the ground penetrating radar module 6, rather the processing module 9 can receive the receive signal 8 from any device which is enabled to provide the receive signal 8 having some data related to the sub-surface 15. For generating the receive signal 8, a transmit signal 7 is first transmitted by a transducer and further the transmit signal 7 on being reflected by the sub-surface 15, a receive signal 8 is generated. The transmit signal 7 and the receive signal 8 can either be based on electromagnetism or acoustics. For acoustics based signals 7, 8, an acoustic transducer and acoustic receiver module, for example microphone, geophone, etc is used and for electromagnetism based signals 7, 8, an electromagnetic based transducer and antenna is used.

The excavations system 1 also includes a material testing unit 10 which tests material of the sub-surface 15 of the environment 17 being excavated. On the basis of the testing, the material testing unit 10 generates a sub-surface test result 11 and provides the sub-surface test result 11 to the processing module 9. The processing module 9 processes the sub-surface test result 11 in combination with the receive signal 8 to generate the sub-surface information 3. In alternate embodiment, the processing module 9 processes the sub-surface test result 11 separately to generate data related the material of the sub-surface 15 which forms an element of the sub-surface information 3.

The ground penetrating radar module 6 sends a transmit signal 7 to a sub-surface 15 of the environment 17 and receives a receive signal 8 from the sub-surface 15 and further transfers the receive signal 8 to the processing module 9. The change in properties between the receive signal 8 and the transmit signal 7 is one of the basis of the generation of sub-surface information 3 by the processing module 9. In an alternate embodiment, ground penetrating radar module 6 need not generate the receive signal 8, rather any other device can be used to generate receive signal 8, capable of transmitting the transmit signal 7 and receiving the receive signal 8 after being reflected by the sub-surface 15.

FIG 2 illustrates the excavator 16 which is a bucket wheel excavator, physically coupled to a ground penetrating radar module 6. Specifically one of the buckets of the bucket wheel excavator 16 has a physical coupling with the ground penetrating module. The ground penetrating radar module 6 sends the transmit signal 7 to the sub-surface 15 of the environment 17 and receives the receive signal after being reflected back by the sub-surface 15. In the FIG 2, only one ground penetrating radar module 6 is shown being used as the physical coupling with excavator 16. So, the receive signal 8 is received only once on every rotation of the wheel of the excavator 16. However, in some critical situation, in other embodiments, the excavator 16 has more than one ground penetrating radar module 6 coupled to on various buckets of the wheel of the excavator 16 at regular intervals of the buckets or irregularly or at all of the wheels of the excavator 16.

In an alternate embodiment, the ground penetrating radar module 6 can be placed in any proximity of the excavator 16.

Depending on the material property, the bucket wheel excavator needs to adjust its frequency. For example, if the material is loose then the excavator can reclaim material faster by increasing it frequency. On the other hand, if the material is denser then the excavator needs to reclaim at slower frequency, so that there is no mechanical fatigue to the system.

While this invention has been described in detail with reference to certain embodiments, it should be appreciated that the present invention is not limited to those precise embodiments. Rather, in view of the present disclosure which describes the current best mode for practicing the invention, many modifications and variations would present themselves, to those of skilled in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. An excavation system (1) comprising:
- a controlling module (2) adapted to receive a sub-surface information (3) of an environment (17) being excavated by an excavator (16) of the excavation system (1), to process the sub-surface information (3) and to send a controlling signal (4) to a drive (5) of the excavator (16), and
- the drive (5) of the excavator (16) adapted to receive the controlling signal (4) from the controlling module (2) and to control the excavator (16) for excavation of the environment (17) on a basis of the controlling signal (4).

2. The excavation system (1) according to the claim 1 comprising:
- a ground penetrating radar module (6) adapted to send a transmit signal (7) to a sub-surface (15) of the environment (17), to receive a receive signal (8) from the sub-surface (15) and to transfer the receive signal (8) to a processing module (9),
- the processing module (9) adapted to receive the receive signal (8) and to process the receive signal (8) to generate the sub-surface information (3).

3. The excavation system (1) according to the preceding claim, wherein the ground penetrating radar module (6) is physically coupled to the excavator (16).

4. The excavation system (1) according to any of the claims 2 or 3, comprising:
- a material testing unit (10) adapted to test material of the sub-surface (15) the environment (17) being excavated and to provide a sub-surface test result (11) to the processing module (9),
Wherein the processing module (9) is adapted to receive the sub-surface test result (11) and to process the sub-surface test result (11) in combination with the receive signal (8) to generate the sub-surface information (3).

5. The excavation system (1) according to any of the claims 2 to 4, comprising:
- a storage (12) adapted to receive the sub-surface information (3), to store the sub-surface information (3) and to provide the sub-surface information (3) to the controlling module (2).

6. The excavation system (1) according to any of the claims 1 to 5, wherein the controlling module (2) is adapted to receive a feedback (13) from a motor (14) of the excavator (16) and to process the feedback (13) in combination with the sub-surface information (3) and to generate the controlling signal (4).

7. The excavation system (1) according to any of the claims from 1 to 6, the controlling signal (4) is at least related to one of a torque to be applied by the drive to the excavator (16) and a frequency of rotation of the excavator (16).

8. The excavation system (1) according any of the claims 1 to 7, wherein the excavator (16) is a bucket wheel excavator (16).

9. A method for excavation comprising:
- receiving and processing of a sub-surface information (3) of an environment (17) being excavated by an excavator (16) by a controlling module (2) to send a controlling signal (4) to a drive (5) of the excavator (16),
- receiving the controlling signal (4) by the drive (5) from the controlling module (2), and
- controlling the excavator (16) by the drive (5) on a basis of the controlling signal (4).

10. The method according to the claim 9 comprising:
- sending a transmit signal (7) to a sub-surface (15) of the environment (17) by a ground penetrating radar module (6),
- receiving a receive signal (8) by the ground penetrating radar module (6) from the sub-surface (15),
- transferring the receive signal (8) from the ground penetrating radar module (6) to a processing module (9), and
- processing the receive signal (8) by the processing module (9) to generate the sub-surface information (3).

11. The method according to the claim 10 comprising:
- testing of a material of the sub-surface (15) of the environment (17) being excavated by a material testing unit (10),
- generating and providing a sub-surface test result (11) by the material testing unit (10) to the processing module (9),
- receiving the sub-surface test result (11) by the processing module (9), and
- processing the sub-surface test result (11) in combination with the receive signal (8) by the processing module (9) to generate the sub-surface information (3).

12. The method according to any of the claims from 9 to 11 comprising:
- receiving a feedback (13) by the controlling module (2) from a motor (14) of the excavator (16),
- processing the feedback (13) in combination with the sub-surface information (3) by the controlling module (2), and
- generating the controlling signal (4) by the controlling module (4) on a basis of such processing.
